# EUROPÄISCHE PATENTSCHRIFT

(11) **EP 2 131 341 B1**
(45) Veröffentlichungstag und Bekanntmachung des Hinweises auf die Patenterteilung: **06.07.2011**
(21) Anmeldenummer: 09006193.8
(22) Anmeldetag: 07.05.2009
(51) Int. Cl.: G08G 1/095, G08G 1/07, A61F 9/08

(54) **Anforderungsgerät für eine Verkehrsampel**
Request device for a traffic light
Appareil de sollicitation pour un feu de circulation

(30) Priorität: 05.06.2008 DE 102008026943
(43) Veröffentlichungstag der Anmeldung: 09.12.2009
(73) Patentinhaber: Langmatz GmbH, 82467 Garmisch-Partenkirchen (DE)
(72) Erfinder:
(74) Vertreter: Flosdorff, Jürgen

(56) Entgegenhaltungen:
- EP-A- 0 414 063
- DE-C1- 10 129 562
- FR-A- 2 632 474

## Beschreibung

Die Erfindung betrifft ein Anforderungsgerät für eine Verkehrsampel, insbesondere für einen Fußgängerübergang, mit einem Schalter, durch dessen Betätigung ein Schaltvorgang auslösbar ist mit der Folge, dass über eine vorbestimmte Zeitdauer ein Freigabe-Lichtsignal, in der Regel das grüne Lichtsignal, erscheint. Das Anforderungsgerät der hier betrachteten Art enthält zudem eine akustische Einrichtung, die während der Grün-Phase einen Freigabe-Ton abgibt, und außerdem einen Vibrator, der während der Grün-Phase durch Ansteuersignale einer Vibrator-Steuereinrichtung in Schwingung versetzt wird, wodurch ein zugehöriger Bereich des Gehäuses des Anforderungsgerätes vibriert, wodurch blinde Verkehrsteilnehmer dann, wenn sie aufgrund von Gehörschäden den Freigabe-Ton nicht oder kaum hören können, die Grün-Phase bei Berührung des Gehäuses über den Tastsinn erfahren können.

Es ist z.B aus der DE 10129562C1 bekannt, dass ein Anforderungsgerät der oben beschriebenen Art zudem mit einer Sensoreinrichtung zur Messung der Umgebungslautstärke versehen sein kann, die entsprechend der erfassten Umgebungslautstärke erzeugte Signale an eine Steuereinrichtung abgibt, die die Lautstärke des Freigabe-Tons entsprechend der Umgebungslautstärke - d.h. hauptsächlich des jeweils auftretenden Verkehrslärms - variiert, so dass der Freigabe-Ton auch dann deutlich zu hören ist, wenn im Bereich der Verkehrsampel laute Motorengeräusche z.B. durch LKW auftreten.

Diese Anpassung der Lautstärke des abgegebenen Freigabe-Tons an den Lärmpegel der Umgebungsgeräusche funktioniert bisher nur unzufriedenstellend, da die Vibration der Vibratormechanik, der umliegenden Komponenten und wenigstens eines Bereichs des Gehäuses des Anforderungsgerätes Geräusche erzeugt, die die Sensoreinrichtung bei der Messung der Umgebungslautstärke mit erfasst und die die Messwerte der in der Nähe angeordneten Sensoreinrichtung verfälschen. Es kommt hinzu, dass der aus der Vibration herrührende Geräuschpegel sich im Laufe der Zeit erheblich verändern kann.

Der vorliegenden Erfindung liegt die Aufgabe zugrunde, ein Anforderungsgerät der betrachteten Art so zu verbessern, dass der von der akustischen Einrichtung abgegebene Freigabe-Ton besser an die jeweils herrschende Umgebungslautstärke anpassbar ist.

Diese Aufgabe wird erfindungsgemäß durch die Merkmale des Patentanspruchs 1 gelöst.

Vorteilhafte Ausgestaltungen der Erfindung sind in den abhängigen Ansprüchen gekennzeichnet.

Die Erfindung sieht vor, dass die Vibrator-Steuereinrichtung über vorbestimmte Zeitspannen der Grün-Phase die Ansteuersignale an den Vibrator abgibt mit dazwischen liegenden Pausen, die zur Messung der Umgebungslautstärke vorgesehen sind, und dass die Vibrator-Steuereinrichtung im Anschluss an das Ende jeder dieser Zeitspannen wenigstens ein, vorzugsweise mehrere gegenphasige Ansteuersignale abgibt, die das Nachschwingen des Vibrators, der umliegenden Komponenten und des Gehäuses dämpfen, wodurch die jeweilige Nachschwingzeit erheblich verkürzt wird. Dies hat zur Folge, dass während einer Grün-Phase in kurzen Abständen von beispielsweise einer Sekunde die tatsächliche jeweilige Umgebungslautstärke gemessen werden kann, ohne dass der Messwert durch Nachschwingen von Komponenten des Anforderungsgerätes verfälscht wird, da dieses Nachschwingen durch ein gegenphasiges Ansteuern des Vibrators schnellstens abgestellt werden kann.

Als ein Beispiel sei angeführt, dass der Vibrator durch geeignete Ansteuersignale 0,8 Sekunden lang in Schwingung versetzt wird, woraufhin er beispielsweise 0,01 Sekunden lang gegenphasig angesteuert wird. Wenn in den folgenden 0,19 Sekunden die Umgebungslautstärke gemessen wird, ergibt sich bei diesem Beispiel ein Rhythmus von jeweils 1 Sekunde, in der Vibration erzeugt, gestoppt und der Lärmpegel gemessen wird.

Die Messwerte der Umgebungslautstärke werden an eine Steuerung der akustischen Einrichtung abgegeben, die entsprechend die Lautstärke des abgegebenen Freigabe-Tons einstellt.

Weiter sieht die Erfindung vor, dass das Anforderungsgerät einen Mikroprozessor mit einer Software enthält, die den Vibrator vorzugsweise über eine Leistungs-Vollbrücke ansteuert. Die Leistungs-Vollbrücke verstärkt die Ansteuersignale des Mikroprozessors und gibt diese an den Vibrator weiter, so dass der Vibrator eine ausreichende mechanische Leistung abgeben kann.

Der Vibrator besteht vorzugsweise im wesentlichen aus einem Elektromagneten, d.h. einer Spule mit einem U-förmigen Eisenkern, der auf einem KunststoffTräger montiert ist, und einer Eisenplatte, die auf einem Kunststoffbügel befestigt ist. Der Bügel ist mit dem Träger über Federn verbunden, die den Bügel von dem Träger weg drücken. Bei Stromfluss durch den Elektromagneten entsteht ein Magnetfeld, das den Bügel mit der Eisenplatte an den Träger heranzieht. Wenn der Stromfluss unterbrochen wird, wird der Bügel von dem Träger durch die Federn wieder weg gedrückt. Durch eine geeignete Ansteuerung des Elektromagneten wird dieser in Schwingung versetzt. Die Schwingfrequenz des Vibrators entspricht der Frequenz (Hüllkurve) des Ansteuersignals. In der ersten halben Periode der Schwingung zieht der Elektromagnet den Bügel heran, der in der zweiten Periode der Schwingung durch die Federn wieder abgestoßen wird. Durch das Schwingen des Vibrators werden auch umliegende Komponenten des Anforderungsgerätes in Schwingung versetzt.

Wenn der Vibrator abgeschaltet wird, schwingt das gesamte System, d.h. die Vibratormechanik, die umliegenden Komponenten und das Gehäuse des Anforderungsgerätes, nach. Eine unmittelbare Messung des Umgebungslärmes nach Abschalten des Vibrators würde durch dieses "Nachschwingen" des Systems verfälscht. Um eine Messung der tatsächlichen Umgebungslautstärke nach Abschalten des Vibrators durchführen zu können, muss mit dem Beginn der Messung gewartet werden, bis das Nachschwingen des Systems abgeklungen ist. Diese Zeitspanne wird durch das gegenphasige Ansteuern des Vibrators im erheblichen Umfang verkürzt.

Weitere Merkmale und Einzelheiten der Erfindung ergeben sich aus der nachfolgenden Beschreibung und den beigefügten Zeichnungen. Dabei zeigen:
- Figur 1: das Schema einer herkömmlichen Ansteuerung des Vibrators;
- Figur 2: das Schema einer erfindungsgemäßen Ansteuerung des Vibrators und
- Figur 3: ein erfindungsgemäßes Prinzipschaltbild der Ansteuerung.

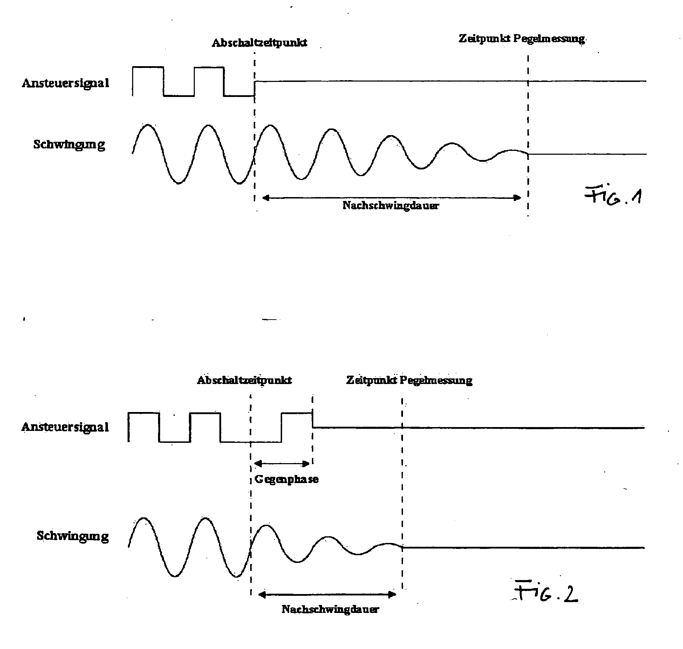

Figur 1 zeigt eine herkömmliche Ansteuerung des Vibrators, bei der die Ansteuersignale zum Abschaltzeitpunkt abgestellt werden. Das System hat eine lange Nachschwingdauer, die nicht zur Messung des tatsächlichen Umgebungslärmpegels genutzt werden kann.
Figur 2 zeigt die erfindungsgemäße Ansteuerung, bei der der Vibrator zum Abschaltzeitpunkt mit gegenphasigen Signalen angesteuert wird. Die Nachschwingdauer ist hierdurch erheblich verkürzt.

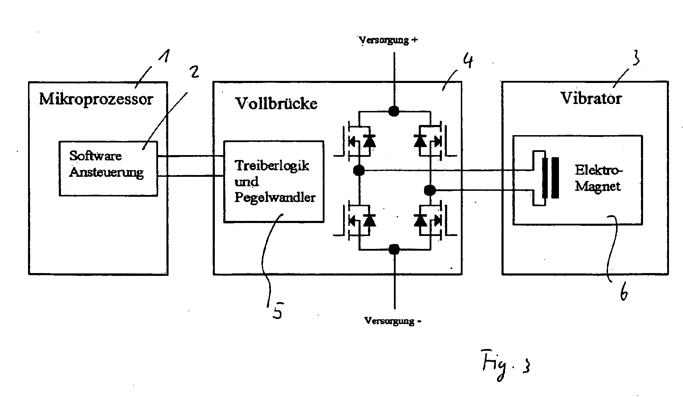

Figur 3 zeigt ein Prinzipschaltbild der erfindungsgemäßen Ansteuerung des Vibrators. In den Mikroprozessor 1 ist die Software 2 implementiert, die den Vibrator 3 über eine Leistungs-Vollbrücke 4 ansteuert. Die Leistungs-Vollbrücke 4 enthält eine Treiberlogik und einen Pegelwandler 5. Der Elektromagnet des Vibrators 3 ist mit dem Bezugszeichen 6 bezeichnet.

Die Ansteuersignale des Mikroprozessors 1 werden in der Vollbrücke 4 verstärkt und an den Vibrator 3 übertragen, so dass der Vibrator 3 eine ausreichende mechanische Leistung abgeben kann.

Es wird betont, dass die Erfindung nicht auf die beschriebenen und dargestellten Ausführungsformen beschränkt ist. Vielmehr sind alle offenbarten Merkmale auf jede Weise einzeln miteinander kombinierbar, sofern diese durch den Schutzbereich der nachfolgenden Ansprüche umfaßt sind.

## Patentansprüche

1. Anforderungsgerät für eine Verkehrsampel, insbesondere für einen Fußgängerübergang, mit einem Schalter, durch dessen Betätigung ein Schaltvorgang auslösbar ist mit der Folge, dass ein Freigabe-Lichtsignal über eine vorbestimmte Zeitdauer erscheint,
mit einem Vibrator (3), der während der Zeitdauer durch Ansteuersignale einer Vibrator-Steuereinrichtung in Schwingung versetzbar ist,
mit einer akustischen Einrichtung, die während der Zeitdauer einen Freigabe-Ton abgibt,
und mit einer Sensoreinrichtung zur Messung der Umgebungslautstärke, wobei die Sensoreinrichtung mit einer Steuereinrichtung für die Lautstärke des abgegebenen Freigabe-Tons verbunden ist,
**dadurch gekennzeichnet,**
**dass** die Vibrator-Steuereinrichtung über vorbestimmte Zeitspannen der Grün-Phase die Ansteuersignale abgibt mit dazwischen liegenden Pausen, in denen die Sensoreinrichtung die Umgebungslautstärke misst, und
**dass** die Vibrator-Steuereinrichtung im Anschluss an das Ende jeder Zeitspanne wenigstens ein, vorzugsweise mehrere gegenphasige Ansteuersignale an den Vibrator abgibt, wodurch die jeweilige Nachschwingzeit verkürzt wird.

2. Anforderungsgerät nach Anspruch 1,
ferner **gekennzeichnet durch**
einen Mikroprozessor (1) mit einer Software (2), die den Vibrator (3) über eine Leistungs-Vollbrücke (4) ansteuert.

3. Anforderungsgerät nach Anspruch 2,
**dadurch gekennzeichnet,**
**dass** die Leistungs-Vollbrücke (4) die Ansteuersignale des Mikroprozessors (1) verstärkt und an den Vibrator (3) überträgt.

4. Anforderungsgerät nach einem der Ansprüche 1 bis 3,
**dadurch gekennzeichnet,**
**dass** der Vibrator (3) einen Elektromagneten, der auf einem vorzugsweise aus Kunststoff bestehenden Träger montiert ist, und eine Eisenplatte aufweist, die auf einem vorzugsweise aus Kunststoff bestehenden Bügel befestigt ist, wobei der Träger mit dem Bügel über Federn verbunden ist, die den Bügel von dem Träger weg drücken.

## Claims

1. A request device for a traffic light, particularly for a pedestrian crossing, including a switch, by whose actuation a switching process may be initiated with the consequence that a clearance light signal appears for a predetermined period of time, a vibrator (3), which may be caused to vibrate during the period of time by control signals from a vibrator control device, an acoustic device, which emits a clearance sound during the period of time, and a sensor device for measuring the ambient noise level, wherein the sensor device is connected to a control device for the volume of the emitted clearance sound, **characterised in that** the vibrator control device supplies the control signals for predetermined time intervals in the green phase with intervening pauses, in which the sensor device measures the ambient noise level and that following the end of each time interval the vibrator control device supplies at least one and preferably a plurality of oppositely phased control signals to the vibrator, whereby the reverberation time is shortened.

2. A request device as claimed in claim 1, further **characterised by** a microprocessor (1) with software (2), which controls the vibrator via a power full bridge (4).

3. A request device as claimed in claim 2, **characterised in that** the power full bridge (4) amplifies the control signals from the microprocessor (1) and transmits them to the vibrator (3).

4. A request device as claimed in one of claims 1 to 3, **characterised in that** the vibrator (3) includes an electromagnet, which is mounted on a support consisting preferably of plastic material, and an iron plate, which is fastened to a bracket consisting preferably of plastic material, wherein the support is connected to the bracket by means of springs, which push the bracket away from the support.

## Revendications

1. Appareil de sollicitation pour un feu de circulation, en particulier pour un passage piétons, avec un commutateur, dont l'actionnement peut déclencher un processus de commutation entraînant l'apparition d'un signal lumineux de libération pendant une durée prédéterminée, comprenant
un vibreur (3) que des signaux de commande d'un dispositif de commande de vibreur peuvent faire vibrer pendant cette durée,
un dispositif acoustique qui délivre un son de libération pendant cette durée,
et un dispositif de détection pour la mesure du volume ambiant, le dispositif de détection étant relié à un dispositif de commande du volume du son de libération délivré,
**caractérisé en ce**
**que** le dispositif de commande de vibreur délivre pendant des intervalles prédéterminés de la phase verte, les signaux de commande avec des pauses au milieu, dans lesquelles le dispositif de détection mesure le volume ambiant, et
en ce que le dispositif de commande de vibreur transmet, à la fin de chaque intervalle, au moins un, de préférence plusieurs signaux de commande en opposition de phase au vibreur, ce qui permet de raccourcir la période de réverbération respective.

2. Appareil de sollicitation selon la revendication 1,
**caractérisé par**
un microprocesseur (1) avec un logiciel (2) commandant le vibreur (3) par un pont intégral de puissance (4).

3. Appareil de sollicitation selon la revendication 2,
**caractérisé en ce**
**que** le pont intégral de puissance (4) amplifie les signaux de commande du microprocesseur (1) et les transmet au vibreur (3).

4. Appareil de sollicitation selon l'une quelconque des revendications 1 à 3,
**caractérisé en ce**
**que** le vibreur (3) présente un électroaimant monté sur un support se composant de préférence de plastique, et une plaque de fer fixée sur un étrier se composant de préférence de plastique, le support étant relié à l'étrier par des ressorts qui repoussent l'étrier du support.
